# NEW EUROPEAN PATENT SPECIFICATION

(11) **EP 0 723 665 B2**
(45) Date of publication and mention of the opposition decision: **02.01.2008**
(45) Mention of the grant of the patent: 28.01.2004
(21) Application number: 95927918.3
(22) Date of filing: 17.08.1995
(51) Int. Cl.: G01N 33/576, G01N 33/50, G01N 33/566

(54) **ASSAY TO DETECT HCV RECEPTOR BINDING**
TEST ZUR ERKENNUNG EINER HCV REZEPTORBINDUNG
TEST POUR DETECTER LA FIXATION DU VIRUS DE L'HEPATITE C A SES RECEPTEURS

(30) Priority: 17.08.1994 GB 9416671
(43) Date of publication of application: 31.07.1996
(73) Proprietor: Novartis Vaccines and Diagnostics S.r.l., 53100 Siena (SI) (IT)
(72) Inventor: ABRIGNANI, Sergio, I-53035 Monteriggioni (IT)
(74) Representative: Hallybone, Huw George
(86) International application number: PCT/IB1995/000692
(87) International publication number: WO 1996/005513

(56) References cited:
- JOURNAL OF EXPERIMENTAL MEDICINE, vol. 178, 1 July 1993 NEW YORK NY USA, pages 17-25, M.A. MINUTELLO ET AL. 'Compartimentalization of T lymphocytes to the cite of disease: intrahepatic CD4+ T cells specific for the protein NS4 of hepatitis C virus in patients with chronic hepatitis C.'
- J. Virol., 1994, vol. 68, p.: 1494-1500
- J. Virol., 1992, vol. 66, p.: 2281-2287
- J. Acquired Immune Defic. Syndrome, 1989, vol. 2, p.: 10-15
- Nature, 1992, vol. 356, p.: 347-350
- J.Virol., 1993, vol. 67, p.: 6025-6032
- PNAS, 1994, vol. 91, p.: 7792-7796

## Description

### Field of the Invention

The present invention relates to an assay to measure binding of hepatitis C virus (HCV) receptor binding ligand, such as HCV proteins, to a target cell receptor. The assay may be used to evaluate vaccine candidates and to identify and measure HCV neutralising antibodies both for research purposes and clinical applications where diagnosing the presence of neutralising antibodies may have a prognostic value in clinical management.

The invention also relates to identifying and characterising the receptor for HCV which will facilitate the identification and screening of antivirals that interfere with receptor interaction.

### Brief Description of the Prior Art

Hepatitis C virus (HCV - previously known as Non-A Non-B hepatitis - NANBV) has become a major health problem world-wide, since 1-2 % of the population is chronically infected with HCV (1). Infection is mostly asymptomatic and resolution occurs in a minority of cases, since 80 to 100 % of the infected individuals become lifelong carriers (2) and chronic hepatitis develops in the majority of these cases (3).

HCV is a positive sense RNA virus of about 10000 nucleotides with a single open reading frame encoding for a polyprotein of about 3000 amino acids. Although the structure of the virus has been elucidated by recombinant DNA techniques (17,18), the virus itself has not been isolated and the functions of the various viral proteins produced by proteolysis of the polyprotein have only been inferred by analogy with other similar viruses of similar genomic organisation.

The viral proteins are all available in recombinant form, expressed in a variety of cells and cell types, including yeast, bacteria, insect and mammalian cells (5,10)

Two proteins, named E1 and E2 (corresponding to amino acids 192-383 and 384-750 respectively) have been suggested to be external proteins of the viral envelope (5) which are responsible for the binding of virus to target cells.

We have devised a method for identifying cells carrying a putative HCV receptor and assay techniques for detecting and quantifying the binding of HCV receptor-binding ligands to the receptor. The technique has a wide range of applications and utilities.

A first step in designing an HCV vaccine is the identification of the components involved in protective immunity. At present, little is known on the role the immune response plays in the course of HCV infection.

The identification of HCV receptor target cells facilitates the characterisation of the HCV receptor itself and provides an important component in the development of assays for binding of HCV receptor-binding ligands to HCV receptor target cells. Such assays may be used in the diagnosis of neutralising antibodies in individuals, the rapid screening of antiviral compounds which interfere with receptor binding and the development of vaccines.

In a passive immunization study in chimpanzees, HCV infection has been prevented after in vitro neutralization with plasma of a chronically infected patient (6). However, the assessment of protective antibody responses to HCV has been hampered by the absence of a neutralization assay *in vitro.* Since HCV does not grow efficiently in cell cultures, attempts have been made (7,8) to set up neutralization tests that estimated HCV binding to target cells. However, the available tests are based on the detection of bound virus by PCR, with obvious shortcomings such as the difficulties in quantitating neutralizing antibodies and problems in obtaining accurate reproduction of RT-PCR testing.

One of these studies is that by Shimizu et al., (1994, J. Virol. 68(3): 1494-1500), who used reverse-transcriptase polymerase chain reaction (RT-PCR) to detect the presence of HCV RNA in infected cells. This diagnostic test was proposed as a test for viral infectivity.

Various literature describes *in vitro* assays for measuring the binding of certain viruses to their target cells, but only in cases where specific receptors for these viruses have been identified. Examples include Kadan et al., (1992, J. Virol. 66 (4): 2281-2287, Schols et al., (1989, Journal of Acquired Immune Deficiency Systems, 2: 10-15), Li et al. (1992, Nature 356: 347-350 and Naniche (1993, J. Virol. 67 (10): 6025-6032). Minutello et al. (1993, J. Exp. Med. 178:17-25) describes a study of the interaction between the non structural 4 (NS4) HCV protein and CD4+ T cells isolated from liver biopsies and measured the binding of NS4 to the T cells using a T cell proliferation assay. However, there is no indication in this document that this mechanism is via an HCV-specific receptor.

The invention also relates to a quantitative test (named Neutralisation of binding or N.O.B.) to estimate HCV neutralizing antibodies which is based on the cytofluorimetric assessment of sera that neutralize the binding of HCV envelope proteins to human cells.

### Summary of the Invention

According to a first aspect of the present invention, there is provided an assay for measuring the binding of an HCV receptor-binding ligand to a target cell comprising the step of:
a) contacting a putative HCV receptor-binding ligand or a competitive binding analogue thereof with an HCV receptor target cell, wherein said contacting comprises:
   i) admixing HCV receptor target cells and a sample to be tested for the presence of HCV receptor-binding ligand to permit binding of any HCV receptor-binding ligand with the HCV receptor target cells;
   ii) removing unbound HCV receptor-binding ligand; and
b) detecting the binding of the ligand or the competitive binding analogue to the HCV receptor target cell using a detectable antibody that is capable of binding to said HCV receptor-binding ligand , wherein said detecting comprises:
   i) admixing with a detectable antibody capable of binding to the HCV receptor-binding ligand to label those HCV receptor target cells which have bound HCV receptor binding ligand; and
   ii) detecting the amount of labelled HCV receptor target cells;
wherein binding of the detectable antibody to said target cell is indicative of a measurement for binding of an HCV receptor-binding ligand or competitive binding analogue thereof to the target cell.

In an alternative embodiment of the first aspect of the invention, there is provided a method for measuring the binding of an HCV receptor-binding ligand to a target cell comprising the steps of:
a) contacting a putative HCV receptor-binding ligand or a competitive binding analogue thereof with an HCV receptor target cell, wherein said contacting comprises:
   i) admixing HCV receptor target cells, a sample to be tested for the presence of HCV receptor-binding ligand and a limiting amount of an HCV receptor-binding ligand analogue to permit competition for binding to the HCV receptor target cells;
   ii) removing unbound HCV receptor-binding ligand; and
b) detecting the binding of the ligand or the competitive binding analogue to the HCV receptor target cell using a detectable antibody that is capable of binding to said HCV receptor-binding ligand, wherein said detecting comprises:
   i) admixing with a detectable antibody capable of binding to the HCV receptor-binding ligand to label those HCV receptor target cells which have bound HCV receptor binding ligand; and
   ii) detecting the amount of HCV receptor target cells bound to the HCV receptor-binding ligand analogue;
wherein binding of the detectable antibody to said target cell is indicative of a measurement for binding of an HCV receptor-binding ligand or competitive binding analogue thereof to the target cell.

Preferably, binding of the HCV receptor-binding ligand or the HCV receptor-binding ligand analogue is detected by labelling bound species and employing a detection system capable of distinguishing between free HCV receptor target cells and bound HCV receptor target cells.

Preferably, the detection system is flow cytometry, more preferably flow cytofluorimetry. In this case, bound species carry a fluorescent label and physical cell separation occurs between labelled and unlabelled cells.

The first aspect of the invention provides a sensitive and fast assay for the ability of a possible HCV receptor-binding ligand to bind to an HCV receptor target cell and facilitates ready screening of possible HCV receptor-binding ligands. Such possible HCV receptor-binding ligands may have utility as antiviral agents or as possible vaccine or assay reagent candidates.

The assay may be a direct binding assay, measuring directly the binding of a HCV receptor-binding ligand to an HCV receptor target cell or may be a competitive assay in which HCV receptor-binding ligand to be measured in a sample competes for binding with an HCV receptor-binding ligand analogue and the amount of HCV receptor-binding ligand analogue is measured.

In a direct binding assay, the assay steps comprise
i) admixing HCV receptor target cells and a sample to be tested for the presence of HCV receptor-binding ligand to permit binding of any HCV receptor-binding ligand with the HCV receptor target cells
ii) removing unbound HCV receptor-binding ligand
iii) admixing with a detectable antibody capable of binding to the HCV receptor-binding ligand to label those HCV receptor target cells which have bound HCV receptor-binding ligand, and
iv) detecting the amount of labelled HCV receptor target cells.

The detectable antibody may be directly labelled or may be detectable by adding a labelled ligand such as an antibody, suitably one specific for the fixed region of the detectable antibody.

The label may be any label capable of directly or indirectly indicating the presence of the label. Preferably, however, the label is a fluorochrome suitable for use in flow cytometry. Suitable such labels include fluoresceinisothiocyanate (FITC), phycoeriphrine (PE) and Texas Red.

The labelled ligand might be any other ligand capable of binding specifically to the detectable antibody. For example, the detectable antibody might itself have covalently-linked biotin and the labelled ligand might be streptavidin.

The detectable antibody may be, for example, a human, rabbit or mouse immunoglobulin such as IgG and the labelled antibody may be a labelled anti-human, anti-rabbit or antimouse antibody.

The detectable antibody or the second labelled antibody may a polyclonal or monoclonal antibody. In either case the antibody may be a binding fragment of an antibody, such as a F(ab') fragment. The monoclonal antibody may be produced by a cell fusion technique or by a recombinant DNA technique such as humanisation or CDR grafting.

In the described example, the detectable antibody is a polyclonal antibody (e.g.rabbit serum) raised against the HCV receptor-binding ligand in an immunised animal host and the second, labelled, antibody is an anti-animal host (e.g. anti-rabbit IgG) antibody labelled with FITC. Preferably however the detectable antibody is a monoclonal antibody.

A control amount of antibody of the same type as the detectable antibody may be added in a parallel experiment as a control. The control amount may be for example a pre-immune serum.

In an indirect binding assay, the assay steps comprise
i) admixing HCV receptor target cells, a sample to be tested for the presence of HCV receptor-binding ligand and a limiting amount of an HCV receptor-binding ligand analogue to permit competition for binding to the HCV receptor target cells,
ii) removing unbound HCV receptor-binding ligand
iii) admixing with a detectable antibody capable of binding to the HCV receptor-binding ligand to label those HCV receptor target cells which have bound HCV receptor-binding ligand, and
iv) detecting the amount of HCV receptor target cells bound to the HCV receptor-binding ligand analogue.

In either case, the amount of labelled HCV receptor target cells may be detected by providing a fluorescent label and performing cell separation using flow cytometry.

According to a second aspect of the invention, there is provided an assay for measuring neutralisation of an HCV receptor binding ligand arising from the binding of a neutralising antibody to an HCV receptor binding ligand (referred to above as an NOB assay) comprising the steps of:
i) admixing HCV receptor target cells, a HCV receptor-binding ligand and a sample to be tested for the presence of an HCV neutralising antibody
ii) removing unbound HCV receptor-binding ligand,
iii) admixing with a detectable antibody capable of binding to the HCV receptor-binding ligand to label those HCV receptor target cells which have bound HCV receptor-binding ligand, and
iv) detecting the amount of HCV receptor target cells bound to the HCV receptor-binding ligand.

In this assay, HCV receptor target cells and HCV neutralising antibodies compete for binding to the HCV receptor-binding ligand.

The second aspect of the invention provides a sensitive and fast assay for identifying antibodies capable of impairing the binding of HCV to HCV receptors. Such antibodies are likely to neutralise the virus and to be one of the effective responses of the immune system arising from a successful immunisation. The level of neutralising antibodies is therefore indicative of the degree of success of an immunisation protocol and neutralising antibodies may themselves be useful as the active principle in pharmaceutical preparations for passive immunisation.

The assay of the second aspect of the invention may also provide a sensitive assay for detecting HCV antibodies in a sample as a method of diagnosis of existing or previous HCV infection. Preferably therefore the sample is a sample from a patient and the invention provides a method for diagnosing an existing or past infection with HCV comprising conducting the assay of the second aspect of the invention on a sample removed from patient.

We have discovered in chimpanzees immunised with E1/E2 produced in HeLa cells that there is a perfect correlation between the presence of neutralising antibodies and protection from a subsequent challenge with homologous HCV. Since the animals with the highest titre remained completely free of virus, we assume that the antibodies are neutralising antibodies induced by the vaccination. Effective neutralisation titres are still present one year after the third boost. Low titres are followed by mild infection which unlike controls was resolved by vaccination.

The capability for measuring neutralising antibodies thus permits diagnosis of past or present HCV infection and has a prognostic value in the treatment of infected patients.

The present assay permits the characterisation of neutralising antibodies and may be used directly as an assay on patient samples or may be used to validate and test reagents for the development of sensitive assays suitable for use in the routine clinical environment. Such assays would include for example enzyme-labelled assays, particularly ELISA.

The assay of the second aspect of the invention may also be useful in identifying a panel of antibodies capable of binding to the HCV receptor enabling mapping of the receptor on HCV receptor target cells.

Finally, the assay of the second aspect of the invention may be useful in determining cross-reactivity between antibodies to HCV external proteins from different HCV genotypes.

The reagents used in the second aspect of the invention may be as described in the first aspect of the invention and the protocols applied *mutatis mutandis*.

According to a third aspect of the present invention, there is provided a method for identifying HCV receptor target cells in a sample of cells comprising the steps of:
i) admixing the sample of cells and an HCV receptor-binding ligand to permit binding of any HCV receptor-binding ligand with any HCV receptor target cells in the sample
ii) removing unbound HCV receptor-binding ligand,
iii) admixing with a detectable antibody capable of binding to the HCV receptor-binding ligand to label those HCV receptor target cells which have bound HCV receptor-binding ligand, and
iv) detecting the amount of labelled HCV receptor target cells.

The reagents used may be as described in the first aspect of the invention and the protocols applied *mutatis mutandis*.

The method of the third aspect of the invention provides a rapid screening technique for cells carrying an HCV receptor. The method may therefore be used to produce the HCV receptor target cell of the first and second aspects of the invention or to produce HCV receptor target cell populations for subsequent analysis and in particular characterisation of the nature and form of the HCV receptor for the purposes of further refining and improving available HCV receptor-binding ligands.

The invention also encompasses products produced or identified using the assays and methods of the invention, including HCV receptor-binding ligands identified using the assays of the invention and vaccine compositions including same. Any development programme involving an assay or method of the present invention falls within the scope of protection sought by this application.

As used herein, the term "HCV receptor target cell" refers to a cell or cell population capable of binding at least one HCV protein. Suitably, the HCV receptor target cell contains at least one HCV protein receptor.

As used herein, the term "HCV receptor-binding ligand" refers to any ligand capable of binding to an HCV receptor. The HCV receptor-binding ligand preferably comprises one or more HCV polypeptides and/or one or more fragments of an HCV polypeptide capable of binding to an HCV receptor. The HCV receptor-binding ligand may be a polypeptide unrelated to HCV yet capable of binding to an HCV receptor. Suitably the HCV receptor-binding ligand is a recombinant HCV protein or a fragment thereof. Such HCV receptor-binding ligands are disclosed in European patent applications EP-A-0318216 and EP-A-0388232. Most preferred are HCV external proteins E1 and E2 or functional equivalents and fragments thereof which retain the ability to bind to an HCV receptor target cell.

Alternatively, the HCV receptor-binding ligand may be non-polypeptide chemical compound capable of binding an HCV receptor. The non-polypeptide chemical compound may be a chemical analogue of a receptor-binding polypeptide, retaining the receptor-binding epitope.

As used herein, the term "HCV receptor-binding ligand analogue" refers to an analogue of an HCV receptor-binding ligand which is capable of cross-reacting with the HCV receptor-binding ligand for binding to the HCV receptor target cell but is distinguishable from the HCV receptor-binding ligand using the assay detection system. Suitably therefore, the HCV receptor-binding ligand analogue is labelled preferably with a fluorescent label either directly or by the further binding of a labelled ligand such as an antibody.

As used herein the term "polypeptide" refers to a sequence of two or more amino acids comprising at least one peptide bond. The amino acids in the sequence may be naturally occurring amino acids or may be synthetic analogues or wholly synthetic amino acids in any mixture or ratio. The term "polypeptide" encompasses generically proteins and modified polypeptides such as naturally or chemically modified polypeptides, including glycoproteins.

As used herein, the term "HCV neutralising antibody" refers to an antibody capable of reducing the interaction between an HCV receptor-binding ligand and a HCV receptor target cell. Preferably the HCV receptor-binding ligand is a native HCV protein. Preferably the HCV neutralising antibody completely prevents binding of a native HCV protein to HCV receptor target cells. HCV neutralising antibodies may be monoclonal antibodies (produced, for example by the Köhler and Milstein technique of cell fusion or by recombinant DNA techniques such as humanisation and CDR grafting) but are preferably polyclonal antibodies, most preferably antibodies produced by the immune system of an immunised host.

### Brief Description of the Drawings

Figure 1 is a schematic diagram illustrating the first aspect of the invention in which HCV receptor-binding ligands bind to receptors on HCV receptor target cells and are measured by first binding rabbit anti-HCV antibody and then by binding a labelled anti-rabbit IgG-FITC F(ab') fragment prior to cell separation by FACScan analysis (See page 14).
Figure 2 is a representation of the results of flow cytometry experiment describing the differential binding of HCV recombinant envelopes expressed in various systems to human cells. Molt-4 cells incubated with medium alone are shown as hatched curves and the open curves represent cells incubated with 5 µg/ml of the indicated HCV recombinant envelopes (See page 16).
Figure 3 shows the binding of E2 expressed in CHO cells to human cells (See page 16).
Figure 4 is a schematic diagram illustrating the second aspect of the invention in which a neutralising antibody prevents an HCV receptor-binding ligand binding to the receptor on a HCV receptor target cell (see page 17).
Figure 5 shows neutralisation of the binding of E2 to target cells by antibodies to the hypervariable region 1 of HCV E2 (see page 19)
Figure 6 shows the neutralisation of E2 binding by HCV target cells by vaccinated chimpanzee sera, demonstrating the correlation between protection and the presence of neutralising antibodies (see page 19).

### Detailed Description of the Invention

The practice of the present invention will employ, unless otherwise indicated, conventional techniques of immunology, cytofluorimetry and molecular biology, which are within the skill of the art. Such techniques are explained fully in the literature (19).

The skilled person will understand and be familiar with the general methods and techniques of assay design and practice. The invention is described herein in sufficient detail for the skilled person to understand and repeat the experiments disclosed.

It will be understood by the skilled person that alterations of the conditions may be necessary to optimise the assay for given HCV receptor-binding ligands, HCV receptor target cells and antibodies.

Standard abbreviations for virus and proteins are used in this specification. Envelope 1 (E1) and Envelope 2 (E2) of HCV refer to the proteins, and fragments thereof, the nucleotide sequence of which are published (17,18). The nucleotides of the E1 and E2 genes and of the encoded proteins vary in different HCV isolates. Therefore, the E1 and E2 for any HCV isolates are identified because included in the amino acid sequences 192-383 and 384-750 respectively.

E1 and E2 have been produced by recombinant DNA techniques using different expression systems (5,10).

### General Materials and Methods

Cells.The human T-cell lymphoma cell line, Molt-4 was obtained from ATCC (Rockville, MD). Cells were expanded with RPMI 1640 (Gibco Laboratories, Grand Island, NY) medium supplemented with 2 mM L-glutamine, 1 % nonessential amino acids, 1mM sodium pyruvate, penicillin (100 units / ml), streptomycin (100 µg / ml), and 10 % (vol / vol) Foetal calf serum (FCS, Gibco).

Recombinant envelope proteins The glycoproteins E1/E2₁₉₉₋₇₄₅ were expressed in HeLa or CHO cells, extracted and purified as described (5,9). E2₃₈₄₋₇₁₅ was expressed and secreted from recombinant CHO cells as described for the truncated E2₆₆₁ (5). For purification of CHO/E2, CHO cells conditioned media was concentrated 15 fold by ultrafiltration, followed by a further 10 fold volume reduction by ammonium sulphate precipitation at 75% saturation, and redissolution into 25 mM Tris-Cl, 1 mM EDTA, pH 7.5; the monoclonal antibody 5E5/H7(specific for CHO/E2) was purified and coupled onto CNBr-activated Sepharose.™ The antibody column was equilibrated in 25 mM Tris-Cl, 0.15 M NaCl, pH 7.5. The ammonium sulphate precipitated E2 was dissolved in 25 mM Tris-Cl, 1 mM EDTA, pH 7.5, and loaded onto the column. The column was washed with PBS plus 1-M NaCl, and then eluted with 3-4 column volumes of Actisep (Sterogene Inc., Arcadia, CA). All of the yellow-coloured Actisep containing fractions were pooled, concentrated in a stirred cell ultrafilter and diafiltered into PBS buffer. E2₃₈₄₋₇₁₅ was expressed and secreted from recombinant Baculovirus (BV) infected cells as described (10). For purification of BV/E2, conditioned medium from insect cells was loaded onto a column of GNA lectin agarose (Vector Laboratories, Burlingame, CA). The column was then washed with PBS plus 0.9 M NaCl, and eluted with 1 M methyl D-mannoside in PBS plus 0.9 M NaCl. The eluate was dialysed against 20 mM potassium phosphate, pH 6, at 4°C. The precipitate, containing mostly contaminants, was removed by centrifugation, and the supernatant loaded onto a column of S-Sepharose Fast Flow™ (Pharmacia, Uppsala, Sweden) equilibrated in 20 mM potassium phosphate, pH 6. The E2 protein was eluted with a gradient to 0.25 M NaCl in 20 mM potassium Phosphate, pH 6. For expression and secretion from yeast of E2 384-715, we used the *Saccharomyces cerevisiae* strain S150-2B and the secretion vector YEpsec1 (11). E2 is secreted as a core glycosylated peptide of 55 KDa. Yeast/E2 was purified by affinity chromatography using a lectin column and the same procedure used for purification of BV/E2 (10). After purification, all the HCV envelope proteins were > 80% pure. ELISA for all antigens were performed according to published procedures (10).

Sera and monoclonal antibodies (mAbs). Rabbit polyclonal antiserum specific for all the envelope proteins described above and sera from chimpanzees that have been immunized with HeLa E1/E2 or with a combination of Yeast/E1₁₉₉₋₃₃₀ and BV/E2₄₀₄₋₆₆₁ (9) were obtained . The monoclonal antibody 291 (IgG1) was obtained from mice immunized with CHO/E2 and screened for the ability to recognise E2 bound to target cells. A synthetic peptide consisting of HCV-1 amino acids 384-414 (E2 hypervariable region 1, HVR1) was coupled through the amino terminal residue to Diphtheria toxoid and used to immunize mice. The mAbs resulting from the fusion were screened by Elisa with overlapping biotinylated 8mer peptides from amino acid 288 to 487 on streptavidin coated plates. An IgG1 mAb (1G2A7) was isolated which recognise in ELISA the epitope 384-414.

### Example 1 - Binding Assay

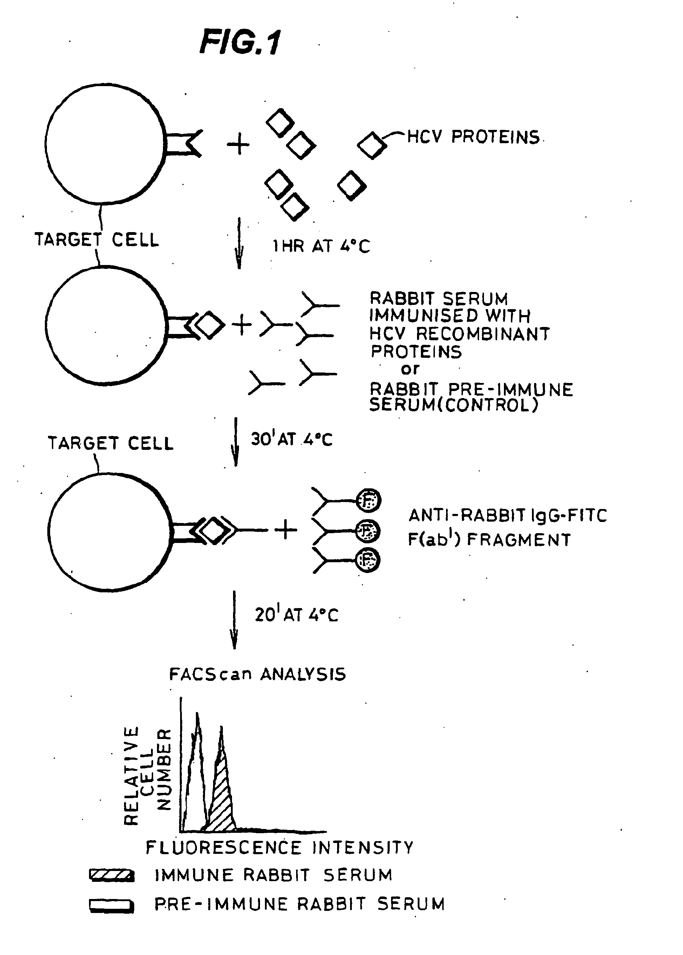

A schematic representation of a binding experiment is shown in Figure 1 which shows the separation achieved by flow cytometric analysis.

An experiment was performed with the aim of measuring the ability of HCV protein to bind to various cell types which should have the putative HCV receptor.

### Experimental Protocol

Indirect immunofluorescence experiments were performed to assess the ability of HCV envelope proteins to bind to Molt-4 cells, which is a human T-cell lymphoma that has been reported to allow a low level of HCV replication in vitro (13).

Cells (10⁵/well) Molt-4 were pelleted in 96 U-bottom microplates by centrifugation at 200 x g for 5 min at 4°C. 20µl of HCV proteins diluted in PBS at different concentrations (from 10 µg/ml to 0.001 µg/ml) were mixed with the pellet of Molt-4 cells and incubated at 4°C for 1 hr. Non-bound HCV proteins were removed by two centrifugations in PBS at 200 x g for 5 min at 4°C. Cells were subsequently incubated for 30 min at 4°C with various dilutions of sera from humans, chimpanzees, or rabbits that had been either infected with HCV or immunized with HCV recombinant proteins; where possible, the corresponding pre-immune sera were used as control. The cells were washed twice in PBS and incubated for 30 minutes with the appropriate dilution of fluorescein-isothiocyanate-conjugated antiserum (either to human IgG or rabbit IgG).

Cells were subsequently washed in PBS at 4°C, resuspended in 100 µl PBS and cell-bound fluorescence was analysed with a FACScan™ flow cytometer (Becton Dickinson, Mountain View, CA). By using a dot plot display of forward and side scatter, the machine is gated to include viable single cells and to exclude cell debris and clumps of cells. A total of 5000 events is collected and analyses of the data is done by using the Lysis II software program from Becton Dickinson. This program produce histograms of each cell sample and calculates the mean channel fluorescence of the cell population, which directly relates to the surface density of fluorescently labelled HCV proteins bound to the cells. Mean fluorescence values (mean channel number) of cells incubated with or without HCV proteins and with immune or preimmmune sera were compared. The threshold for positivity is set for each experiment by flow cytometric analysis of cells without HCV proteins bound which have been incubated with antisera to HCV proteins and the FITC labelled second antibody.

The experiment described above shows that binding of E2 to target cells is measurable and has high affinity.

An experiment was conducted to compare the ability of different HCV proteins expressed in various systems to bind various cell types possessing an HCV receptor.

Cells were incubated with HCV recombinant envelopes (E1/E2 or E2), expressed either in yeast, insect cells or mammalian cells (HeLa or CHO), and subsequently incubated with polyclonal sera from rabbits that have been immunized with the corresponding recombinant proteins. After incubation with FITC-conjugated antiserum to rabbit IgG, the binding of HCV proteins was indirectly detected by flow cytometry as cell-bound fluorescence.

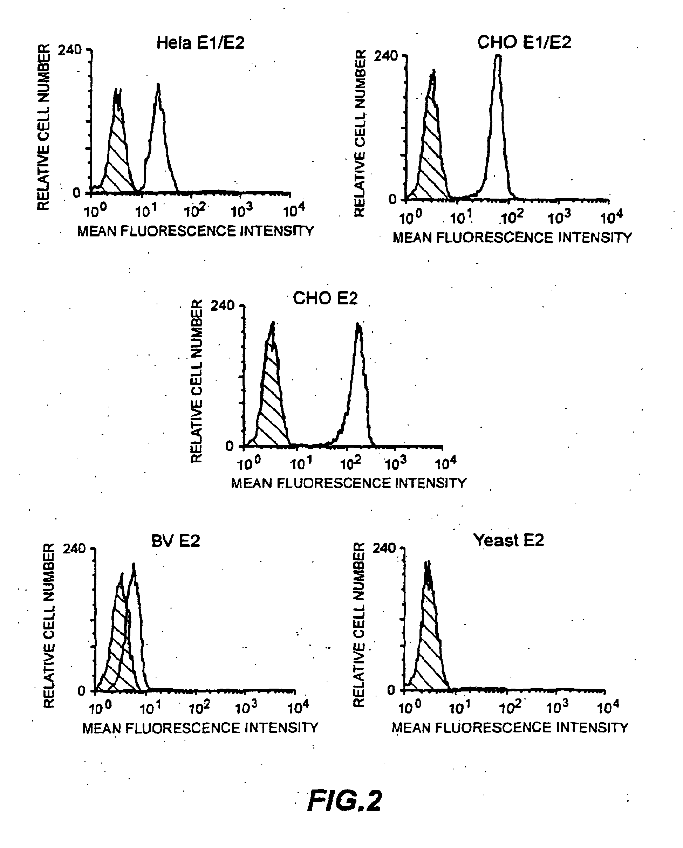

The representative experiments in Figure 2 show that recombinant E1/E2 or E2 expressed in mammalian cells, but not in yeast, can bind human cells, whereas E2 expressed in insect cells has a low, but detectable binding. Identical data were also obtained using as target cells hepatocarcinoma cell lines or freshly purified human B cells.

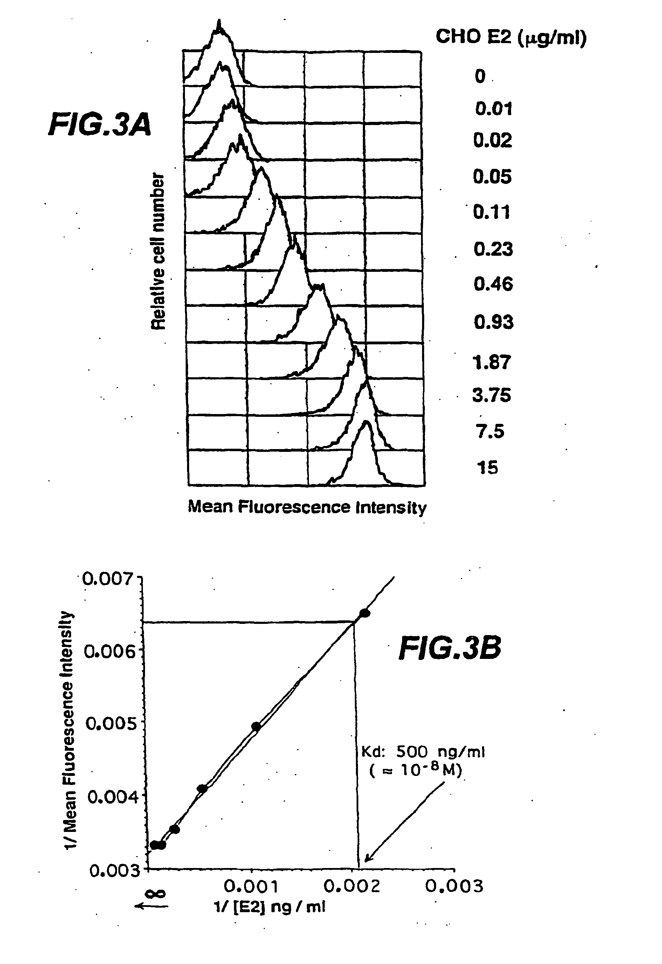

After incubation of the target MOLT-4 cells with increasing concentrations of E1/E2 or E2, it was found (Figure 3A) that the binding of E2 expressed in mammalian cells plateaued at a concentration of 10 µg/ ml.

Since this binding is saturable, the affinity of recombinant E2 for its putative receptor could be estimated using the double reciprocal plot method previously described for the calculation of the affinity of hapten-antibody interaction (14). In Figure 3B, the estimated affinity is expressed as Kd and it is equal to the reciprocal of the free E2 concentration at which half the concentration of E2 is bound to its putative receptor. In the y-axis, the neat mean fluorescence intensity values for each concentration of E2 was calculated by subtracting the mean fluorescence obtained with rabbit anti-E2 serum and FITC-goat anti-rabbit in the absence of E2 from that obtained in the presence of E2. The neat mean fluorescence intensity (y-axis) and the E2 concentration (x-axis) were plotted as reciprocal values.

The Kd of E2 for target cells is about 10⁻⁸M leading to the conclusion that E2 is probably the protein responsible for the specific binding of the E1/E2 complexes to target cells.

### Example 2 - Neutralisation Assay

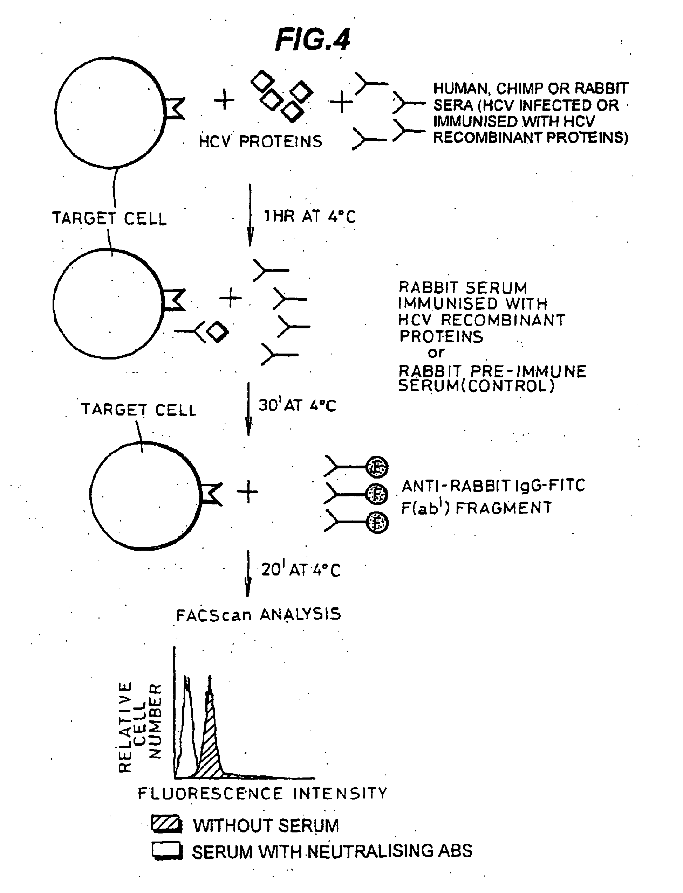

### A schematic representation of a neutralisation assay according to the invention is shown in Figure 4.

### Experimental Protocol

Cells (10⁵/well) from were pelleted in 96 U-bottom microplates by centrifugation at 200 x g for 5 min at 4°C. 20µl of CHO/E2₇₁₅ (0.5 µg/ml PBS) were mixed with various dilutions of sera from humans, chimpanzees or rabbits that are either infected with HCV or have been immunized with HCV recombinant proteins. After incubation at 4°C for 1 hr, Molt-4 cells were added and incubated for 1 hr at 4°C. Non-bound HCV proteins and antibodies were removed by two centrifugations in PBS at 200 x g for 5 min at 4°C. Cells were subsequently incubated for 30 min at 4°C with 1/100 dilution of sera, from the same species of the neutralizing serum, from animals that have been immunized with HCV-envelope recombinant proteins or the corresponding pre-immune sera as control. Revealing the binding with antibodies from the same species of the neutralizing serum is critical, since non-neutralizing anti-E2 antibodies could cover E2 after it is bound to target cells and could therefore interfere with assessment of neutralization if the binding were revealed with an anti-E2 serum from a different species. The cells were washed twice in PBS and incubated for 30 min with the appropriate dilutions of FITC-conjugated antiserum to IgG. Cell-bound fluorescence is analysed as described for the binding assay above.

This technique is very helpful to measure cross-neutralisation of antibodies to HCV envelope proteins from various HCV genotypes. For instance, antibodies raised against envelope proteins from HCV genotype 1A can be assessed for their ability to neutralise binding of Envelope proteins from HCV genotype 1b, 2, 3 etc.

### Antibodies to the E2 Hypervariable Region Neutralise Binding

An experiment was conducted to test whether the binding measured according to Example 1 is neutralisable with antibody to E2. Rabbit polyclonal antisera specific for the recombinant E2 expressed in CHO cells, was assessed for ability to neutralize binding of E2.

E2 (at concentration of 0.5 µg/ml ,i. e., the Kd) was mixed with serial dilutions of the rabbit antisera. The E2-antibody mixture was then incubated with target cells, and the binding of E2 was subsequently detected. It was shown that sera from rabbits immunized with E2 expressed in mammalian cells can neutralize binding of E2 to target cells.

Since for other viruses, epitopes able to induce neutralizing antibodies have been located in regions showing a high degree of variability, further experiments investigated whether a mAb against the HCV-E2 hypervariable region 1(aa 384-414) neutralized binding of E2.

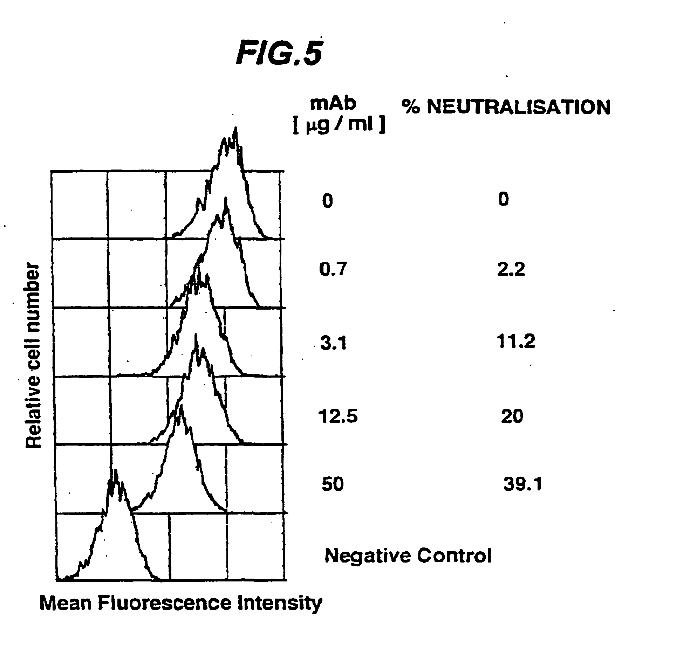

CHO/E2 was preincubated with the indicated concentrations of the purified mAb (1G2A7) specific for HCV-E2 hypervariable region 1 (HVR1). The antibody-E2 mixture was then incubated with Molt-4 cells and the binding was revealed using monoclonal antibody 291 which recognises E2 bound to target cells. Mean fluorescence intensity (MFI) values in the absence of neutralizing mAb (positive control), in the absence of E2 (negative control) and in the presence of E2-antibody complexes (experimental values) were measured and specific neutralization was determined according to the equation: Specific neutralization = x 100 [( positive control MFI - experimental MFI) / (positive control MFI - negative control MFI)]. The results are presented in Figure 5.

Figure 5 shows that the HVR1 specific mAb can neutralize, though not completely binding of E2 demonstrating that binding of E2 is at least in part mediated by hypervariable regions.

Antibodies that neutralize binding of HCV envelope correlate with protection from infection

It has been shown that vaccination with recombinant envelope proteins expressed in mammalian cells (HeLa), but not in yeast or insect cells, could protect chimpanzees from primary infection by an homologous HCV isolate (9).

To investigate whether the binding, and subsequent neutralization, of E2 were relevant to the binding of HCV to target cells, the neutralizing titres present in the sera of chimpanzees vaccinated and protected from subsequent challenge were compared with the sera from chimpanzees immunized but susceptible to HCV challenge.

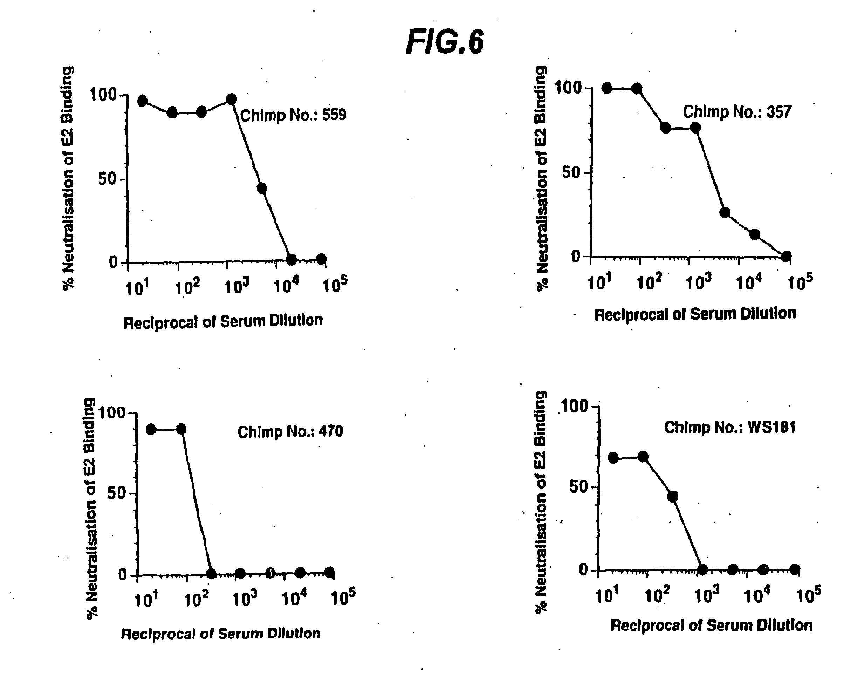

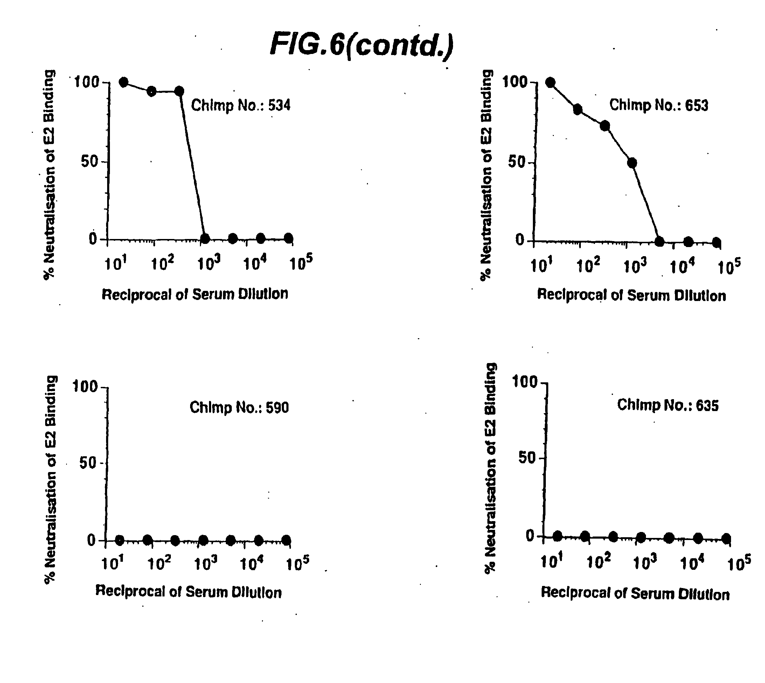

The results are depicted in Figure 6 in which the results for each chimp were as follows:

| | |
|---|---|
| Chimp | 559 |
| Vaccine | HELA E1/E2 |
| Outcome | Protected |
| Anti-E1/E2 ELISA Titre | 1/37900 |
| Anti-E2 HV ELISA Titre | 1/49 |
| 50% Neutralisation Titre | 1/3500 |

| | |
|---|---|
| Chimp | 357 |
| Vaccine | HELA E1/E2 |
| Outcome | Protected |
| Anti-E1/E2 ELISA Titre | 1/25900 |
| Anti-E2 HV ELISA Titre | 1/30 |
| 50% Neutralisation Titre | 1/2500 |

| | |
|---|---|
| Chimp | 534 |
| Vaccine | HELA E1/E2 |
| Outcome | Protected |
| Anti-E1/E2 ELISA Titre | 1/22300 |
| Anti-E2 HV ELISA Titre | 1/64 |
| 50% Neutralisation Titre | 1/600 |

| | |
|---|---|
| Chimp | 653 |
| Vaccine | HELA E1/E2 |
| Outcome | Protected |
| Anti-E1/E2 ELISA Titre | 1/8700 |
| Anti-E2 HV ELISA Titre | ND |
| 50% Neutralisation Titre | 1/1500 |

| | |
|---|---|
| Chimp | 470 |
| Vaccine | HELA E1/E2 |
| Outcome | Infected but resolved |
| Anti-E1/E2 ELISA Titre | 1/5300 |
| Anti-E2 HV ELISA Titre | 1/95 |
| 50% Neutralisation Titre | 1/250 |

| | |
|---|---|
| Chimp | WS181 |
| Vaccine | HELA E1/E2 |
| Outcome | Infected but resolved |
| Anti-E1/E2 ELISA Titre | 1/2600 |
| Anti-E2 HV ELISA Titre | 1/ND |
| 50% Neutralisation Titre | 1/300 |

| | |
|---|---|
| Chimp | 590 |
| Vaccine | Yeast E1/BV E2 |
| Outcome | Infected |
| Anti-E1/E2 ELISA Titre | 1/4300 |
| Anti-E2 HV ELISA Titre | ND |
| 50% Neutralisation Titre | 1/0 |

| | |
|---|---|
| Chimp | 635 |
| Vaccine | Yeast E1/BV E2 |
| Outcome | Infected |
| Anti-E1/E2 ELISA Titre | 1/2800 |
| Anti-E2 HV ELISA Titre | 1/42 |
| 50% Neutralisation Titre | 1/0 |

Figure 6 and the above data show that all chimpanzees that had NOB neutralizing titres of at least 1/600 were protected from infection, chimpanzees with NOB titres of about 1/300 developed a mild infection and resolved, whereas chimpanzees with no NOB antibodies were not protected (9).

Serial dilutions of sera from chimpanzees vaccinated with recombinant envelope proteins (9) were tested for their ability to neutralize binding of E2. In each square is indicated the envelope proteins used as vaccine, the outcome of challenge with HCV-1 containing plasma, the ELISA titres against HeLa E1/E2 and the peptide corresponding to the E2-HVR1, and the 50% neutralization titres calculated as in Figure 5.

Figure 6 also shows that ELISA titres to E2-hypervariable region 1 were comparable in protected versus non-protected chimpanzees demonstrating that E2-binding neutralizing antibodies correlate with protection from infection, and that neutralization induced by vaccination does not depend on antibodies to the HVR1.

In parallel, sera from human infected with a given HCV genotype can be tested for ability to neutralise binding of envelope proteins from HCV genotypes different from the infecting genotype.

It will be understood that the invention is described above by way of example and modifications may be made without the need for undue experiment or the exercise of inventive ingenuity.

### References

1. Van der Poel, C. L. et al (1994) Lancet 344, 1475-1479.
2. Alter, M.J., et al (1992) N. Engl. J.Med. 327 1899-1905.
3. Alter, M.J., et al (1989) in Current Perspectives in Hepatology, eds Seef, L.B. et al (Plenum, New York) 83-97.
4. Choo, Q. -L. et al (1991) Proc. Natl. Acad. Sci. USA 88, 2451-2455.
5. Spaete, R. R. et al (1992) Virology 188, 819-830.
6. Farci, P. et al (1994) Proc. Natl. Acad. Sci. USA 91, 7792- 7796.
7. Shimizu, Y. K. et al (1994) J. Virol. 68, 1494- 1500.
8. Zibert, A. et al (1995) Virol. 208, 653- 661.
9. Choo, Q. -L. et al (1994) Proc. Natl. Acad. Sci. USA 91, 1294-1298.
10. Chien, D. Y. et al (1992) Proc. Natl. Acad. Sci. USA 89, 10011-10015.
11. Baldari, C. et al (1987) EMBO J. 6, 229-234.
12. Lau, J. Y. N. et al (1995) J. Infect. Dis. 171, 281-289.
13. Shimizu, Y. K. et al (1992) Proc. Natl. Acad. Sci. USA 89, 5477-5481.
14. Celada, F. et al (1973) Immunochemistry 10 797-804.
15. Weiner, A. J. et al (1992) Proc. Natl. Acad. Sci. USA 89, 3468-3472.
16. Botarelli, P. et al (1993) Gastroenterology 104 580-587
17. European patent application EP-A-0318216
18. European patent application EP-A-0388232
19. Virology, Eds Dulbecco R. et al Harper & Row Philadelphia 1980.

## Claims

1. A method for measuring the binding of an HCV receptor-binding ligand to a target cell comprising the steps of:
a) contacting a putative HCV receptor-binding ligand or a competitive binding analogue thereof with an HCV receptor target cell, wherein said contacting comprises:
i) admixing HCV receptor target cells and a sample to be tested for the presence of HCV receptor-binding ligand to permit binding of any HCV receptor-binding ligand with the HCV receptor target cells;
ii) removing unbound HCV receptor-binding ligand; and
b) detecting the binding of the ligand or the competitive binding analogue to the HCV receptor target cell using a detectable antibody that is capable of binding to said HCV receptor-binding ligand, wherein said detecting comprises:
i) admixing with a detectable antibody capable of binding to the HCV receptor-binding ligand to label those HCV receptor target cells which have bound HCV receptor binding ligand; and
ii) detecting the amount of labelled HCV receptor target cells;
wherein binding of the detectable antibody to said target cell is indicative of a measurement for binding of an HCV receptor-binding ligand or competitive binding analogue thereof to the target cell.

2. A method for measuring the binding of an HCV receptor-binding ligand to a target cell comprising the steps of:
a) contacting a putative HCV receptor-binding ligand or a competitive binding analogue thereof with an HCV receptor target cell, wherein said contacting comprises:
i) admixing HCV receptor target cells, a sample to be tested for the presence of HCV receptor-binding ligand and a limiting amount of an HCV receptor-binding ligand analogue to permit competition for binding to the HCV receptor target cells;
ii) removing unbound HCV receptor-binding ligand; and
b) detecting the binding of the ligand or the competitive binding analogue to the HCV receptor target cell using a detectable antibody that is capable of binding to said HCV receptor-binding ligand, wherein said detecting comprises:
i) admixing with a detectable antibody capable of binding to the HCV receptor-binding ligand to label those HCV receptor target cells which have bound HCV receptor binding ligand; and
ii) detecting the amount of HCV receptor target cells bound to the HCV receptor-binding ligand analogue;
wherein binding of the detectable antibody to said target cell is indicative of a measurement for binding of an HCV receptor-binding ligand or competitive binding analogue thereof to the target cell.

3. A method for measuring neutralisation of an HCV receptor binding ligand arising from the binding of a neutralising antibody to an HCV receptor binding ligand comprising the steps of:
i) admixing HCV receptor target cells, a HCV receptor binding ligand and a sample to be tested for the presence of an HCV neutralising antibody;
ii) removing unbound HCV receptor-binding ligand;
iii) admixing with a detectable antibody capable of binding to the HCV receptor-binding ligand to label those HCV receptor target cells which have bound HCV receptor binding ligand; and
iv) detecting the amount of HCV receptor target cells bound to the HCV receptor-binding ligand.

4. A method for identifying HCV receptor target cells in a sample of cells comprising the steps of:
i) admixing the sample of cells and an HCV receptor binding ligand to permit binding of any HCV receptor binding ligand with any HCV receptor target cells in the sample;
ii) removing unbound HCV receptor-binding ligand;
iii) admixing with a detectable antibody capable of binding to the HCV receptor-binding ligand to label those HCV receptor target cells which have bound HCV receptor binding ligand; and
iv) detecting the amount of labelled HCV receptor target cells.

5. A method according to any one of the preceding claims wherein the amount of HCV receptor target cells bound to the HCV receptor-binding ligand is detected by flow cytometry.

6. A method for identifying neutralising binding antibodies to an HCV receptor comprising the step of performing a method as defined in claim 3.

## Patentansprüche

1. Verfahren zur Messung der Bindung eines HCV-Rezeptorbindungsliganden an eine Zielzelle, das die Schritte umfasst:
a) Inkontaktbringen eines putativen HCV-Rezeptorbindungsliganden oder eines kompetitiven Bindungsanalogons davon mit einer HCV-Rezeptorzielzelle, wobei das Inkontaktbringen umfasst:
i) Mischen von HCV-Rezeptorzielzellen und einer Probe, die auf die Anwesenheit eines HCV-Rezeptorbindungsliganden getestet werden soll, um das Binden eines beliebigen HCV-Rezeptorbindungsliganden mit den HCV-Rezeptorzielzellen zu ermöglichen;
ii) Entfernen von ungebundenem HCV-Rezeptorbindungsliganden; und
b) Nachweisen der Bindung des Liganden oder des kompetitiven Bindungsanalogons an die HCV-Rezeptorzielzelle unter Verwendung eines nachweisbaren Antikörpers, der in der Lage ist, an den HCV-Rezeptorbindungsliganden zu binden, wobei der Nachweis umfasst:
i) Mischen mit einem nachweisbaren Antikörper, der in der Lage ist, an den HCV-Rezeptorliganden zu binden, um die HCV-Rezeptorzielzellen, die einen HCV-Rezeptorbindungsliganden gebunden haben, zu markieren; und
ii) Feststellen der Menge an markierten HCV-Rezeptorzielzellen;
wobei die Bindung des nachweisbaren Antikörpers an die Zielzelle auf eine Messung der Bindung eines HCV-Rezeptorbindungsliganden oder eines kompetitiven Bindungsanalogons davon an die Zielzelle hinweist.

2. Verfahren zur Messung der Bindung eines HCV-Rezeptorbindungsliganden an eine Zielzelle, das die Schritte umfasst:
a) Inkontaktbringen eines putativen HCV-Rezeptorbindungsliganden oder eines kompetitiven Bindungsanalogons davon mit einer HCV-Rezeptorzielzelle, wobei das Inkontaktbringen umfasst:
i) Mischen von HCV-Rezeptorzielzellen, einer Probe, die auf die Anwesenheit eines HCV-Rezeptorbindungsliganden getestet werden soll, und einer begrenzten Menge eines HCV-Rezeptorbindungsligandenanalogons, um Konkurrenz um die Bindung an die HCV-Rezeptorzielzellen zu ermöglichen;
ii) Entfernen von ungebundenem HCV-Rezeptorbindungsliganden; und
b) Nachweisen der Bindung des Liganden oder des kompetitiven Bindungsanalogons an die HCV-Rezeptorzielzelle unter Verwendung eines nachweisbaren Antikörpers, der in der Lage ist, an den HCV-Rezeptorbindungsliganden zu binden, wobei der Nachweis umfasst:
i) Mischen mit einem nachweisbaren Antikörper, der in der Lage ist, an den HCV-Rezeptorbindungsliganden zu binden, um die HCV-Rezeptorzielzellen, die einen HCV-Rezeptorbindungsliganden gebunden haben, zu markieren; und
ii) Feststellen der Menge an HCV-Rezeptorzielzellen, die an das HCV-Rezeptorbindungsligandenanalogon gebunden haben;
wobei die Bindung des nachweisbaren Antikörpers an die Zielzelle auf eine Messung der Bindung eines HCV-Rezeptorbindungsliganden oder eines kompetitiven Bindungsanalogons davon an die Zielzelle hinweist.

3. Verfahren zur Messung der Neutralisation eines HCV-Rezeptorbindungsliganden, die aus der Bindung eines neutralisierenden Antikörpers an einen HCV-Rezeptorbindungsliganden hervorgeht, das die Schritte umfasst:
i) Mischen von HCV-Rezeptorzielzellen, eines HCV-Rezeptorbindungsliganden und einer Probe, die auf die Anwesenheit eines HCV neutralisierenden Antikörpers getestet werden soll;
ii) Entfernen von ungebundenem HCV-Rezeptorbindungsliganden;
iii) Mischen mit einem nachweisbaren Antikörper, der in der Lage ist, an den HCV-Rezeptorbindungsliganden zu binden, um die HCV-Rezeptorzielzellen, die einen HCV-Rezeptorbindungsliganden gebunden haben, zu markieren; und
iv) Feststellen der Menge an HCV-Rezeptorzielzellen, die an den HCV-Rezeptorbindungsliganden gebunden sind.

4. Verfahren zur Identifizierung von HCV-Rezeptorzielzellen in einer Zellprobe, das die Schritte umfasst:
i) Mischen der Zellprobe und eines HCV-Rezeptorbindungsliganden, um das Binden eines beliebigen HCV-Rezeptorbindungsliganden mit beliebigen HCV-Rezeptorzielzellen in der Probe zu ermöglichen;
ii) Entfernen von ungebundenem HCV-Rezeptorbindungsliganden;
iii) Mischen mit einem nachweisbaren Antikörper, der in der Lage ist, an den HCV-Rezeptorbindungsliganden zu binden, um die HCV-Rezeptorzielzellen, die einen HCV-Rezeptorbindungsliganden gebunden haben, zu markieren; und
iv) Feststellen der Menge an markierten HCV-Rezeptorzielzellen.

5. Verfahren nach einem der vorangehenden Ansprüche, wobei die Menge an HCV-Rezeptorzielzellen, die an die HCV-Rezeptorbindungsliganden gebunden sind, mittels Durchflusscytometrie festgestellt wird.

6. Verfahren zur ldentifizierung neutralisierender Bindungsantikörper zu einem HCV-Rezeptor, das den Schritt der Ausführung eines Verfahrens, wie in Anspruch 3 definiert, umfasst.

## Revendications

1. Procédé de mesure de la liaison d'un ligand de liaison au récepteur du VHC à une cellule cible comprenant les étapes consistant à :
a) mettre en contact un ligand de liaison au récepteur du VHC putatif ou un analogue de liaison compétitif de celui-ci avec une cellule cible récepteur du VHC, ladite mise en contact comprenant:
i) le mélange de cellules cibles récepteurs du VHC et d'un échantillon à tester pour la présence de ligand de liaison au récepteur du VHC en vue de permettre la liaison de tout ligand de liaison au récepteur du VHC aux cellules cibles récepteurs du VHC ;
ii) l'élimination de ligand de liaison au récepteur du VHC non lié ; et
b) détecter la liaison du ligand ou de l'analogue de liaison compétitif à la cellule cible récepteur du VHC en utilisant un anticorps susceptible d'être détecté et capable de se lier audit ligand de liaison au récepteur du VHC, ladite détection comprenant:
i) le mélange avec un anticorps susceptible d'être détecté et capable de se lier au ligand de liaison au récepteur du VHC afin de marquer ces cellules cibles récepteurs du VHC qui se sont liées au ligand de liaison au récepteur du VHC ; et
ii) la détection de la quantité de cellules cibles récepteurs du VHC marquées ;
dans lequel la liaison de l'anticorps susceptible d'être détecté à ladite cellule cible est indicative d'une mesure de liaison d'un ligand de liaison au récepteur du VHC ou d'un analogue de liaison compétitif de celui-ci à la cellule cible.

2. Procédé de mesure de la liaison d'un ligand de liaison au récepteur du VHC à une cellule cible comprenant les étapes consistant à :
a) mettre en contact un ligand de liaison au récepteur du VHC putatif ou un analogue de liaison compétitif de celui-ci avec une cellule cible récepteur du VHC, ladite mise en contact comprenant :
i) le mélange des cellules cibles récepteurs du VHC, d'un échantillon à tester pour la présence de ligand de liaison au récepteur du VHC et d'une quantité limitante d'un analogue du ligand de liaison au récepteur du VHC en vue de permettre une compétition pour la liaison aux cellules cibles récepteurs du VHC ;
ii) l'élimination de ligand de liaison au récepteur du VHC non lié ; et
b) détecter la liaison du ligand ou de l'analogue de liaison compétitif à la cellule cible récepteur du VHC en utilisant un anticorps susceptible d'être détecté et capable de se lier audit ligand de liaison au récepteur du VHC, ladite détection comprenant:
i) le mélange avec un anticorps susceptible d'être détecté et capable de se lier au ligand de liaison au récepteur du VHC afin de marquer les cellules cibles récepteurs du VHC qui se sont liées au ligand de liaison au récepteur du VHC ; et
ii) la détection de la quantité de cellules cibles récepteurs du VHC liées à l'analogue du ligand de liaison au récepteur du VHC ;
dans lequel la liaison de l'anticorps susceptible d'être détecté à ladite cellule cible est indicative d'une mesure de liaison d'un ligand de liaison au récepteur du VHC ou d'un analogue de liaison compétitif de celui-ci à la cellule cible.

3. Procédé de mesure de la neutralisation d'un ligand de liaison au récepteur du VHC provenant de la liaison d'un anticorps neutralisant à un ligand de liaison au récepteur du VHC comprenant les étapes consistant à :
i) mélanger des cellules cibles récepteurs du VHC, un ligand de liaison au récepteur du VHC et un échantillon à tester pour la présence d'un anticorps neutralisant le VHC ;
ii) éliminer le ligand de liaison au récepteur du VHC non lié ;
iii) mélanger avec un anticorps susceptible d'être détecté et capable de se lier au ligand de liaison au récepteur du VHC afin de marquer les cellules cibles récepteurs du VHC qui se sont liées au ligand de liaison au récepteur du VHC ; et
iv) détecter la quantité de cellules cibles récepteurs du VHC liées au ligand de liaison au récepteur du VHC.

4. Procédé d'identification de cellules cibles récepteurs du VHC dans un échantillon de cellules comprenant les étapes consistant à :
i) mélanger l'échantillon de cellules et un ligand de liaison au récepteur du VHC en vue de permettre la liaison de tout ligand de liaison au récepteur du VHC à toutes cellules cibles récepteurs du VHC dans l'échantillon ;
ii) éliminer le ligand de liaison au récepteur du VHC non lié ;
iii) mélanger avec un anticorps susceptible d'être détecté et capable de se lier au ligand de liaison au récepteur du VHC afin de marquer les cellules cibles récepteurs du VHC qui se sont liées au ligand de liaison au récepteur du VHC ; et
iv) détecter la quantité de cellules cibles récepteurs du VHC marquées.

5. Procédé selon l'une quelconque des revendications précédentes, dans lequel la quantité de cellules cibles récepteurs du VHC liées au ligand de liaison au récepteur du VHC est détectée par cytométrie de flux.

6. Procédé d'identification d'anticorps de liaison neutralisants à un récepteur du VHC comprenant l'étape consistant à effectuer un procédé tel que défini dans la revendication 3.
